(19) Europäisches Patentamt
European Patent Office
Office européen des brevets

(11) EP 4 371 660 A1

(12) EUROPEAN PATENT APPLICATION

(43) Date of publication:
22.05.2024 Bulletin 2024/21

(21) Application number: 22208496.4

(22) Date of filing: 21.11.2022

(51) International Patent Classification (IPC):
$B01J\ 8/00^{(2006.01)}$    $B01J\ 8/08^{(2006.01)}$
$B01J\ 8/10^{(2006.01)}$    $C08F\ 220/18^{(2006.01)}$
$C09J\ 133/06^{(2006.01)}$

(52) Cooperative Patent Classification (CPC):
(C-Sets available)
B01J 8/0015; B01J 8/005; B01J 8/007;
B01J 8/085; B01J 8/10; C07C 67/39; C08F 20/14;
B01J 8/226; B01J 2208/00575; B01J 2208/00584;
B01J 2208/00646      (Cont.)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: Röhm GmbH
64295 Darmstadt (DE)

(72) Inventor: The designation of the inventor has not
yet been filed

(74) Representative: Röhm Patent Association
IP Management
Deutsche-Telekom-Allee 9
64295 Darmstadt (DE)

(54) **METHOD FOR PREPARING A CATALYST FOR THE PRODUCTION OF METHYL METHACRYLATE USING CLARIFICATION**

(57) A method for providing a reactor at least partially filled with a reactant mass, comprising the steps of

a. providing a reactor that has an inner volume, wherein the reactor comprises

i. at least one agitation means, preferably located in a first zone of the inner volume;

ii. at least one liquid-solid separation means, wherein the at least one liquid-solid separation means

A. comprises at least one outlet that is at least partially closed, and

B. is located in the inner volume, preferably in a further zone of the inner volume;

b. at least partially filling the inner volume with a first mixture, wherein

i. the volume of the first mixture defines a pre-reactant mass, and

ii. the first mixture comprises

A. a particulated porous catalyst, and

B. a first liquid;

c. agitating the first mixture in the inner volume with the at least one agitation means,

i. thereby obtaining a circulation of the first mixture in the inner volume,

ii. wherein

A. preferably a surface loading rate of the first mixture through the at least one liquid-solid separation means is $V_1$,

B. preferably a temperature in the inner volume is $T_1$, and

C. preferably a pressure in the inner volume is $P_1$;

d. at least partially opening the at least one outlet to allow at least a partial volume of the first mixture to exit the inner volume through the at least one outlet,

i. thereby obtaining the reactant mass, wherein the reactant mass is defined as the volume of the first mixture remaining in the inner volume, and

ii. wherein preferably the surface loading rate of the first mixture through the at least one liquid-solid separation means is $V_2$.

(52)  Cooperative Patent Classification (CPC): (Cont.)

C-Sets
**C07C 67/39, C07C 69/54**

**Description**

**FIELD OF THE INVENTION**

The inventions pertains to a method for providing a reactor at least partially filled with a reactant mass, comprising the steps of

a. providing a reactor that has an inner volume, wherein the reactor comprises

i. at least one agitation means, preferably located in a first zone of the inner volume;
ii. at least one liquid-solid separation means, wherein the at least one liquid-solid separation means

A. comprises at least one outlet that is at least partially closed, and
B. is located in the inner volume, preferably in a further zone of the inner volume;

b. at least partially filling the inner volume with a first mixture, wherein

i. the volume of the first mixture defines a pre-reactant mass, and
ii. the first mixture comprises

A. a particulated porous catalyst, and
B. a first liquid;

c. agitating the first mixture in the inner volume with the at least one agitation means,

i. thereby obtaining a circulation of the first mixture in the inner volume,
ii. wherein

A. preferably a surface loading rate of the first mixture through the at least one liquid-solid separation means is $V_1$,
B. preferably a temperature in the inner volume is $T_1$, and
C. preferably a pressure in the inner volume is $P_1$;

d. at least partially opening the at least one outlet to allow at least a partial volume of the first mixture to exit the inner volume through the at least one outlet,

i. thereby obtaining the reactant mass, wherein the reactant mass is defined as the volume of the first mixture remaining in the inner volume, and
ii. wherein preferably the surface loading rate of the first mixture through the at least one liquid-solid separation means is $V_2$.

The inventions also pertains to the reactant mass, as well as a method for producing a product stream using the reactant mass.

BACKGROUND

Methyl methacrylate is a widely used chemical product, with several million tonnes produced worldwide every year. Various production processes are known for producing methyl methacrylate, as summarised in Krill, Offermanns, and Rühling (2019), *Chem. Unserer Zeit,* **53**. One example of these processes is the socalled $C_2$ process wherein ethylene is converted into propionaldehyde via a hydroformylation reaction and the propionaldehyde is converted into methacrolein via a Mannich Kondensation type reaction with formaldehyde. The resulting methacrolein is then, together with methanol, directly oxidatively esterified into methyl methacrylate and water as couple product. The oxidative esterification step is catalysed by a particulated porous catalyst.
The size distribution of the particles making up the particulated porous catalyst has an influence on the yield of a product stream (comprising, *e.g.*, methyl methacrylate). For example, a smaller $D_{90}$ of the particles making up the particulated porous catalyst generally increase the yield, compared to a larger $D_{90}$. It is therefore preferred to have $D_{90}$ below a certain value. However, a smaller $D_{90}$ is accompanied by a smaller $D_1$. Therefore, a larger number of very small particles (sizes smaller than 20 $\mu$m) will form part of the particulated porous catalyst. These very small particles are generally carried out of the reactor (in which the product stream is produced) along with the product stream. As a result, the very small particles very quickly clog any filter used to filter the product stream, thus requiring that the filters be cleaned more regularly. This cleaning of the filters is time and energy intensive, and significantly reduces the throughput in the production of the product stream.

A method is thus required to remove the very small particles of the particulated porous catalyst. However, current methods for removing the very small particles are also very time and energy intensive, and generally fail to remove a sufficient number of the very small particles.

## OBJECTS

An object of the present invention is to at least partially overcome at least one of the disadvantages encountered in the state of the art.

It is a further object of the invention to provide a method for increasing the $D_1$ value of the particles that make up a particulated porous catalyst, by *e.g.*, removing particles that have a size of less than 20 $\mu$m, wherein the method leads to a larger increase in the $D_1$ value.

It is a further object of the invention to provide a method for increasing the $D_1$ value of the particles that make up a particulated porous catalyst, by *e.g.*, removing particles that have a size of less than 20 $\mu$m, wherein the method requires less time.

It is a further object of the invention to provide a method for increasing the $D_1$ value of the particles that make up a particulated porous catalyst, by *e.g.*, removing particles that have a size of less than 20 $\mu$m, wherein the method requires less energy.

It is a further object of the invention to provide a method for increasing the $D_1$ value of the particles that make up a particulated porous catalyst, by *e.g.*, removing particles that have a size of less than 20 $\mu$m, wherein the method requires less equipment.

It is a further object of the invention to provide a method for increasing the $D_1$ value of the particles that make up a particulated porous catalyst without increasing the $D_{90}$ value of said particles by more than 10 %.

It is a further object of the invention to provide a method for at least partially removing particles, that form part of a particulated porous catalyst and that have a size of less than 20 $\mu$m, from a reactor that is used to produce a product stream.

It is a further object of the invention to provide a method that increases the filter lifetime of a filter used to filter a product stream.

It is a further object of the invention to provide a method that reduces the amount of a particulated porous catalyst in a product stream, wherein said catalyst is used for the production of the product stream.

It is a further object of the invention to provide a method that reduces the amount of a particulated porous catalyst lost during the production of a product stream, wherein said catalyst is used for the production of the product stream.

It is a further object of the invention to provide a method for producing a product stream, wherein said method has an increased throughput.

It is a further object of the invention to provide a reactor that is at least partially filled with a reactant mass, wherein the reactant mass is used in the production of a product stream, and wherein the reactant mass comprises a particulated porous catalyst that has a $D_1 > 30$ $\mu$m. The particulated porous catalyst is used during the production of the product stream.

It is a further object of the invention to provide a reactor that is at least partially filled with a reactant mass, wherein the reactant mass is used in the production of a product stream, and wherein the reactant mass comprises a particulated porous catalyst that has a $D_{90} < 110$ $\mu$m. The particulated porous catalyst is used during the production of the product stream.

It is a further object of the invention to provide a product stream that has a higher purity, *e.g.*, comprises less catalyst.

It is a further object of the invention to provide a polymer that has a higher purity, *e.g.*, comprises less catalyst.

## PREFERRED EMBODIMENTS OF THE INVENTION

A contribution to at least partially fulfilling at least one of the above-mentioned objects is made by any of the embodiments of the invention.

A 1st embodiment of the invention is a method for providing a reactor at least partially filled with a reactant mass, comprising the steps of

    a. providing the reactor that has an inner volume, wherein the reactor comprises

i. at least one agitation means, preferably located in a first zone of the inner volume;
ii. at least one liquid-solid separation means, wherein the at least one liquid-solid separation means

    A. comprises at least one outlet that is at least partially closed, and
    B. is located in the inner volume, preferably in a further zone of the inner volume;

b. at least partially filling the inner volume with a first mixture, wherein

    i. the volume of the first mixture defines a pre-reactant mass, and
    ii. the first mixture comprises

        A. a particulated porous catalyst, and
        B. a first liquid;

c. agitating the first mixture in the inner volume with the at least one agitation means,

    i. thereby obtaining a circulation of the first mixture in the inner volume,
    ii. wherein

        A. preferably a surface loading rate of the first mixture through the at least one liquid-solid separation means is $V_1$,
        B. preferably a temperature in the inner volume is $T_1$, and
        C. preferably a pressure in the inner volume is $P_1$;

d. at least partially opening the at least one outlet to allow at least a partial volume of the first mixture to exit the inner volume through the at least one outlet,

    i. thereby obtaining the reactant mass, wherein the reactant mass is defined as the volume of the first mixture remaining in the inner volume, and
    ii. wherein preferably the surface loading rate of the first mixture through the at least one liquid-solid separation means is $V_2$.

In an aspect of the 1st embodiment, it is preferred that the outlet in step a. is closed. In an aspect of the 1st embodiment, it is preferred that the value of $V_1$ does not exceed the value of $V_2$ by more than 10 %. In an aspect of the 1st embodiment, it is preferred that $V_1 = 0$ m$^3$/(h·m$^2$). The *"at least partially opening"* of the at least one outlet in step d. of the 1st embodiment should preferably be understood to mean that the flow rate of a liquid through the at least one outlet is increased. In an aspect of the 1st embodiment, it is preferred that the first mixture is also agitated during step d. In an aspect of the 1st embodiment, it is preferred the first liquid is a reactant used to obtain a product stream. In an aspect of the 1st embodiment, it is preferred that the temperature in the inner volume during step d. is $T_1$. In an aspect of the 1st embodiment, it is preferred that the pressure in the inner volume during step d. is $P_1$. In an aspect of the 1st embodiment, it is preferred that the reactor is at least partially filled with additional first liquid after the at least one outlet has been opened (step d.). In this aspect, it is preferred that the additional first liquid is added continually, batch-wise, or both. In this aspect, it is preferred that at least a partial volume of the additional first liquid that is added is allowed to exit the inner volume through the at least one outlet.

In an aspect of the 1st embodiment, it is preferred that the first mixture comprises an even-further liquid. In this aspect, it is preferred that the density of the even-further liquid is larger than the density of the first liquid, more preferably at least 1.05 times larger, even more preferably at least 1.1 times larger, and further preferably at least 1.2 times larger. In the aspect wherein the first mixture comprises an even-further liquid, it is preferred that prior to obtaining the first mixture (by mixing the particulated porous catalyst, the first liquid, and the even-further liquid), the particulated porous catalyst is first mixed with the even-further liquid to obtain an even-further mixture, followed by mixing the even-further mixture with the first liquid to obtain the first mixture. In this aspect, it is preferred that the even-further mixture is mixed with the first liquid at least 30 minutes, more preferably at least 1 hour, and further preferably at least 6 hours after mixing the particulated porous catalyst with the even-further liquid to obtain the even-further mixture. In the aspect wherein the first mixture comprises an even-further liquid, it is preferred that the even-further liquid is a is a reactant used to obtain a product stream.

In a preferred embodiment of the method for providing a reactor at least partially filled with a reactant mass, less than 10 vol-%, preferably less than 5 vol-%, and more preferably less than 1 vol-% of the first liquid in the inner volume is converted to a different chemical substance, chemical composition, or both, by a chemical reaction, where the vol-% is based on a total volume of the first liquid in the inner volume. This preferred embodiment is a 2nd embodiment of the invention, that preferably depends on the 1st embodiment of the invention.

In a preferred embodiment of the method for providing a reactor at least partially filled with a reactant mass, a filling density of the particulated porous catalyst is at least 1.2 times, preferably at least 1.8 times, and more preferably at least 2.0 times higher than a density of the first liquid in the inner volume. This preferred embodiment is a 3rd embodiment of the invention, that preferably depends on any of the 1st to 2nd embodiments of the invention.

In a preferred embodiment of the method for providing a reactor at least partially filled with a reactant mass, the particulated porous catalyst has at least one or all of the following properties:

a. the filling density of the particulated porous catalyst is in the range of 1 $g/cm^3$ to 3.6 $g/cm^3$, preferably in the range of 1.3 $g/cm^3$ to 3 $g/cm^3$, and more preferably in the range of 1.7 $g/cm^3$ to 2.3 $g/cm^3$;
b. the density of the first liquid in the inner volume is in the range of 0.3 $g/cm^3$ to 1.9 $g/cm^3$, preferably in the range of 0.5 $g/cm^3$ to 1.7 $g/cm^3$, and more preferably in the range of 0.6 $g/cm^3$ to 1.2 $g/cm^3$.

This preferred embodiment is a 4th embodiment of the invention, that preferably depends on any of the 1st to 3rd embodiments of the invention.

In an aspect of the 4th embodiment, all possible combination of the features a. and b. are preferred aspects of the embodiment. These combinations are *e.g.,* a; b; a+b. In a preferred aspect of the 4th embodiment, the density of the first liquid in the inner volume is temperature dependent. In this aspect, it is preferred that the temperature of the first liquid in the inner volume is controlled such that the first liquid has a density that falls within at least one of the ranges of feature b. in the 4th embodiment. In a preferred aspect of the 4th embodiment, the density of the first liquid in the inner volume is pressure dependent. In this aspect, it is preferred that the pressure in the inner volume is controlled such that the first liquid has a density that falls within at least one of the ranges of feature b. in the 4th embodiment.

In a preferred embodiment of the method for providing a reactor at least partially filled with a reactant mass, the particulated porous catalyst has at least one or all of the following:

a. an envelope density in the range of 0.5 $g/cm^3$ to 2.8 $g/cm^3$, preferably in the range of 0.9 $g/cm^3$ to 2.3 $g/cm^3$, and more preferably in the range of 1.3 $g/cm^3$ to 1.9 $g/cm^3$;
b. a true density in the range of 1.9 $g/cm^3$ to 4.4 $g/cm^3$, preferably in the range of 2.4 $g/cm^3$ to 3.9 $g/cm^3$, and more preferably in the range of 2.9 $g/cm^3$ to 3.5 $g/cm^3$.

This preferred embodiment is a 5th embodiment of the invention, that preferably depends on any of the 1st to 4th embodiments of the invention.

In an aspect of the 5th embodiment, all possible combination of the features a. and b. are preferred aspects of the embodiment. These combinations are *e.g.,* a; b; a+b.

In a preferred embodiment of the method for providing a reactor at least partially filled with a reactant mass, the first liquid in the inner volume has a dynamic viscosity in the range of 0.01 mPa·s to 2.1 mPa·s, preferably in the range of 0.05 mPa·s to 1.8 mPa·s, and more preferably in the range of 0.1 mPa·s to 1.5 mPa·s. This preferred embodiment is a 6th embodiment of the invention, that preferably depends on any of the 1st to 5th embodiments of the invention.

In a preferred aspect of the 6th embodiment, the dynamic viscosity of the first liquid in the inner volume is temperature dependent. In this aspect, it is preferred that the temperature in the inner volume is controlled such that the first liquid has a dynamic viscosity that falls within at least one of the ranges of the 6th embodiment. In a preferred aspect of the 6th embodiment, the dynamic viscosity of the first liquid in the inner volume is pressure dependent. In this aspect, it is preferred that the pressure in the inner volume is controlled such that the first liquid has a dynamic viscosity that falls within at least one of the ranges of the 6th embodiment.

In a preferred embodiment of the method for providing a reactor at least partially filled with a reactant mass, the at least one liquid-solid separation means is a lamella clarifier. This preferred embodiment is a 7th embodiment of the invention, that preferably depends on any of the 1st to 6th embodiments of the invention.

In a preferred embodiment of the method for providing a reactor at least partially filled with a reactant mass, $V_1 < V_2$. This preferred embodiment is an 8th embodiment of the invention, that preferably depends on any of the 1st to 7th embodiments of the invention.

In a preferred embodiment of the method for providing a reactor at least partially filled with a reactant mass, at least one or all of the following applies:

a. during agitation of the first mixture in the inner volume (step c.), the surface loading rate of the first mixture through the at least one liquid-solid separation means, $V_1$, is less than 0.05 $m^3/(h·m^2)$, preferably less than 0.01 $m^3/(h·m^2)$, and more preferably less than 0.001 $m^3/(h·m^2)$;
b. after opening of the at least one outlet to allow the at least a partial volume of the first mixture to exit the inner volume through the at least one outlet (step d.), the surface loading rate of the first mixture through the at least one liquid-solid separation means, $V_2$, is in the range of 0.2 m³/(h·m²) to 2 m³/(h·m²), preferably in the range of 0.25 m³/(h·m²) to 1.5 m³/(h·m²), and more preferably in the range of 0.5 m³/(h·m²) to 1.0 m³/(h·m²).

This preferred embodiment is a 9th embodiment of the invention, that preferably depends on any of the 1st to 8th embodiments of the invention.

In an aspect of the 9th embodiment, all possible combination of the features a. and b. are preferred aspects of the embodiment. These combinations are *e.g.,* a; b; a+b.

In a preferred embodiment of the method for providing a reactor at least partially filled with a reactant mass, the method further comprises the step of feeding a first gaseous feed stream into the inner volume. This preferred embodiment is a 10th embodiment of the invention, that preferably depends on any of the 1st to 9th embodiments of the invention.

In an aspect of the 10th embodiment, it is preferred that the first gaseous feed stream is fed into the inner volume at least partially simultaneously while performing at least one or all of the following steps in the 1st embodiment: step b., step c., and step d.

In a preferred embodiment of the method for providing a reactor at least partially filled with a reactant mass, the first gaseous feed stream comprises at least one or all of the following:

   a. in the range of 40 vol-% to 100 vol-%, preferably in the range of 50 vol-% to 100 vol-%, and more preferably in the range of 60 vol-% to 100 vol-% nitrogen;
   b. $\leq$ 21 vol-%, preferably $\leq$ 15 vol-%, and more preferably $\leq$ 10 vol-% oxygen.

This preferred embodiment is an 11th embodiment of the invention, that preferably depends on the 10th embodiment of the invention.

The vol-% in the 11th embodiment are based on a total volume of the first gaseous feed. In an aspect of the 11th embodiment, all possible combination of the features a. and b. are preferred aspects of the embodiment. These combinations are *e.g.,* a; b; a+b.

In a preferred embodiment of the method for providing a reactor at least partially filled with a reactant mass, the first mixture is agitated using at least one or all of the following:

   a. a physical agitation means, *e.g.,* an impeller, a stirrer, a turbine, a propeller;
   b. the first gaseous feed stream;
   c. a first liquid feed stream;
   d. a recirculation pump;
   e. a recirculation compressor, preferably adapted and arranged for the recirculation of a gas flow.

This preferred embodiment is a 12th embodiment of the invention, that preferably depends on any of the 1st to 11th embodiments of the invention.

In an aspect of the 12th embodiment, all possible combination of the features a. to e. are preferred aspects of the embodiment. These combinations are *e.g.,* a; b; c; d; e; a+b; a+c; a+d; a+e; b+c; b+d; b+e; c+d; c+e; d+e; a+b+c; a+b+d; a+b+e; a+c+d; a+c+e; a+d+e; b+c+d; b+c+e; b+d+e; c+d+e; a+b+c+d; a+b+c+e; a+b+d+e; a+c+d+e; b+c+d+e; a+b+c+d+e.

In a preferred embodiment of the method for providing a reactor at least partially filled with a reactant mass, a mass fraction of particles forming part of the particulated porous catalyst in the inner volume and with a particle size $\leq$ 75 $\mu$m, preferably with a size $\leq$ 50 $\mu$m, and more preferably with a size $\leq$ 25 $\mu$m, is reduced. This preferred embodiment is a 13th embodiment of the invention, that preferably depends on any of the 1st to 12th embodiments of the invention.

In the 13th embodiment, the mass fraction is based on the total mass of the particulated porous catalyst in the inner volume. In order to determine the reduction in the mass fraction in the 13th embodiment, the mass fraction is measured prior to opening the at least one outlet (prior to step d. in the 1st embodiment). The mass fraction is then measured again after opening the at least one outlet (step d. in the 1st embodiment) and after at least 10 vol-% of the first mixture has exited the reactor. The vol-% is based on the volume of the first mixture prior to opening the at least one outlet.

In a preferred embodiment of the method for providing a reactor at least partially filled with a reactant mass, a ratio $D_1/D_{90}$ of the particulated porous catalyst in the inner volume is increased by a factor of at least 1.2, preferably at least 1.5, and further preferably at least 2. This preferred embodiment is a 14th embodiment of the invention, that preferably depends on any of the 1st to 13th embodiments of the invention.

In the 14th embodiment, the increase in the ratio $D_1/D_{90}$ is determined as follows:

$$\text{Increase in } D_1/D_{90} = (D_1/D_{90})^{\text{after}} / (D_1/D_{90})^{\text{before}}$$

where $(D_1/D_{90})^{\text{before}}$ is measured prior to opening the at least one outlet (prior to step d. in the 1st embodiment), and $(D_1/D_{90})^{\text{after}}$ is measured after opening the at least one outlet (step d. in the 1st embodiment) and after at least 10 vol-% of the first mixture has exited the reactor. The vol-% is based on the volume of the first mixture prior to opening the at least one outlet.

In a preferred embodiment of the method for providing a reactor at least partially filled with a reactant mass, the particles forming part of the particulated porous catalyst present in the inner volume has at least one or all of the following properties:

a. prior to opening the at least one outlet, $D_1$ is in the range of 1 $\mu$m to 40 $\mu$m, preferably in the range of 5 $\mu$m to 35 $\mu$m, and more preferably in the range of 10 $\mu$m to 30 $\mu$m;
b. prior to opening the at least one outlet, $D_{90}$ is in the range of 75 $\mu$m to 130 $\mu$m, preferably in the range of 85 $\mu$m to 120 $\mu$m, and more preferably in the range of 95 $\mu$m to 110 $\mu$m;
c. prior to opening the at least one outlet, the ratio $(D_1/D_{90})^{\text{before}}$ is in the range of 0.06 to 0.32, preferably in the range of 0.12 to 0.26, and more preferably in the range of 0.16 to 0.22;
d. after opening the at least one outlet, $D_1$ is in the range of 10 $\mu$m to 80 $\mu$m, preferably in the range of 15 $\mu$m to 60 $\mu$m, and more preferably in the range of 20 $\mu$m to 50 $\mu$m;
e. after opening the at least one outlet, $D_{90}$ is in the range of 78 $\mu$m to 133 $\mu$m, preferably in the range of 88 $\mu$m to 123 $\mu$m, and more preferably in the range of 98 $\mu$m to 113 $\mu$m;
f. after opening the at least one outlet, the ratio $(D_1/D_{90})^{\text{after}}$ is in the range of 0.28 to 0.6, preferably in the range of 0.34 to 0.50, and more preferably in the range of 0.38 to 0.46.

This preferred embodiment is a 15th embodiment of the invention, that preferably depends on any of the 1st to 14th embodiments of the invention.

In the 15th embodiment, feature a., $D_1$ is measured prior to opening the at least one outlet (prior to step d. in the 1st embodiment). In the 15th embodiment, feature b., $D_1$ is measured after opening the at least one outlet (step d. in the 1st embodiment) and after at least 10 vol-% of the first mixture has exited the reactor. In the 15th embodiment, $(D_1/D_{90})^{\text{before}}$ in feature c. is measured prior to opening the at least one outlet (prior to step d. in the 1st embodiment). In the 15th embodiment, $D_1$ (feature d.), $D_{90}$ (feature e.), and $(D_1/D_{90})^{\text{after}}$ (feature f.) are measured after opening the at least one outlet (step d. in the 1st embodiment) and after at least 10 vol-% of the first mixture has exited the reactor. The vol-% is based on the volume of the first mixture prior to opening the at least one outlet. In an aspect of the 15th embodiment, all possible combination of the features a. to f. are preferred aspects of the embodiment. These combinations are *e.g.,* a; b; c; d; e; f; a+b; a+c; a+d; a+e; a+f; b+c; b+d; b+e; b+f; c+d; c+e; c+f; d+e; d+f; e+f; a+b+c; a+b+d; a+b+e; a+b+f; a+c+d; a+c+e; a+c+f; a+d+e; a+d+f; a+e+f; b+c+d; b+c+e; b+c+f; b+d+e; b+d+f; b+e+f; c+d+e; c+d+f; c+e+f; d+e+f; a+b+c+d; a+b+c+e; a+b+c+f; a+b+d+e; a+b+d+f; a+b+e+f; a+c+d+e; a+c+d+f; a+c+e+f; a+d+e+f; b+c+d+e; b+c+d+f; b+c+e+f; b+d+e+f; c+d+e+f; a+b+c+d+e; a+b+c+d+f; a+b+c+e+f; a+b+d+e+f; a+c+d+e+f; b+c+d+e+f; a+b+c+d+e+f.

In a preferred embodiment of the method for providing a reactor at least partially filled with a reactant mass, at least one or all of the following applies:

a. the temperature $T_1$ is in the range of 10 °C to 110 °C, more preferably in the range of 25 °C to 80 °C, and further preferably in the range of 40 °C to 50 °C;
b. the pressure $P_1$ is in the range of 0.5 bar absolute to 9 bar absolute , preferably in the range of 4.5 bar absolute to 8.5 bar absolute , and more preferably in the range of 6 bar absolute to 8 bar absolute .

This preferred embodiment is a 16th embodiment of the invention, that preferably depends on any of the 1st to 15th embodiments of the invention.

In an aspect of the 16th embodiment, all possible combinations of the features a. and b. are preferred aspects of the embodiment. These combinations are *e.g.,* a; b; a+b. In an aspect of the 16th embodiment, it is preferred that feature a., feature b., or both, also apply during step d. in the 1st embodiment.

In a preferred embodiment of the method for providing a reactor at least partially filled with a reactant mass, the first mixture comprises at least one or all of the following:

a. in the range of 0.01 wt-% to 30 wt-%, preferably in the range of 0.05 wt-% to 25 wt-%, and more preferably in the range of 0.1 wt-% to 20 wt-% of the particulated porous catalyst;
b. in the range of 30 wt-% to 99.99 wt-%, preferably in the range of 40 wt-% to 80 wt-%, and more preferably in the range of 50 wt-% to 75 wt-% of the first liquid.

This preferred embodiment is a 17th embodiment of the invention, that preferably depends on any of the 1st to 16th embodiments of the invention.

In the 17th embodiment, the wt-% are based on a total weight of the first mixture prior to opening the at least one outlet in step d. of the 1st embodiment. In an aspect of the 17th embodiment, all possible combination of the features a. and b. are preferred aspects of the embodiment. These combinations are *e.g.,* a; b; a+b.

In a preferred embodiment of the method for providing a reactor at least partially filled with a reactant mass, the first liquid comprises water, an organic compound, or both. This preferred embodiment is an 18th embodiment of the invention, that preferably depends on any of the 1st to 17th embodiments of the invention. In an aspect of the 18th embodiment, a preferred organic compound is a $C_1$ to $C_8$ organic compound, more preferably an alcohol, an ester, an organic acid, a ketone, an ether, an alkane, or a combination of at least two or more thereof. In this aspect, a preferred alcohol is methanol, ethanol, propanol, butanol, wherein methanol and ethanol are more preferred and methanol is most preferred. In this aspect, a preferred ester is a meth-alkyl ester, wherein methyl methacrylate is most preferred. In particular preferred is the combination of methanol and methyl methacrylate. A preferred ketone is an acetone. A preferred ether is dimethoxyethane.

In a preferred embodiment of the method for providing a reactor at least partially filled with a reactant mass, the particulated porous catalyst comprises one element from the 10th group and a further element from the 11th group of the periodic table, preferably in the form of an oxide, wherein the oxide preferably comprises at least one other element selected from the 1st, 2nd, 13th and 14th groups of the periodic table, or a combination of at least two thereof. This preferred embodiment is a 19th embodiment of the invention, that preferably depends on any of the 1st to 18th embodiments of the invention.

In an aspect of the 19th embodiment, it is preferred that the catalyst comprises at least nickel, cobalt, gold, or a combination of at least two thereof, preferably in form of a metal oxide, or at least one mixed metal oxide, or a combination thereof. A preferred catalyst according to the invention is at least one or all of the following: an $Au/NiO/SiO_2$-$Al_2O_3$- MgO powder catalyst, an $Au/CO_3O_4/SiO_2$-$Al_2O_3$-MgO (or any other cobalt oxides) catalyst, and a gold catalyst where the nickel/cobalt is at least partially replaced by at least one Lanthanide.

In a preferred embodiment of the method for providing a reactor at least partially filled with a reactant mass, at least one or all of the following applies:

  a. the partial volume of the first mixture that exits through the at least one outlet is in the range of 1 vol% to 70 vol-%, preferably in the range of 5 vol% to 60 vol-%, and more preferably in the range of 10 vol% to 50 vol-%, based on the volume of the first mixture prior to opening the at least one outlet;
  b. the partial volume of the first mixture that exits through the at least one outlet leads to a reactor turn-around time that is in the range of 1 hour to 3 hours, preferably in the range of 1.5 hour to 2.8 hours, and more preferably in the range of 1.7 hour to 2.3 hours.

This preferred embodiment is a 20th embodiment of the invention, that preferably depends on any of the 1st to 19th embodiments of the invention.

In an aspect of the 20th embodiment, all possible combination of the features a. and b. are preferred aspects of the embodiment. These combinations are *e.g.,* a; b; a+b. The *"turn-around time"* in feature b. of the 20th embodiment should preferably be understood as the time required to complete an exchange of the inner volume. E.g., for an inner volume of 400 $m^3$ and a surface loading rate through the at least one liquid-solid separation means of 200 $m^3$/h, the turn-around time is 2 hours.

In a preferred embodiment of the method for providing a reactor at least partially filled with a reactant mass, during the step of opening the at least one outlet (step d. in the 1st embodiment), $\leq$ 15 wt-%, preferably $\leq$ 5 wt-%, more preferably $\leq$ 3 wt-%, and further preferably $\leq$ 1 wt-% of the particulated porous catalyst in the inner volume is removed from the inner volume when the at least partial volume of the first mixture exits the inner volume through the at least one outlet. This preferred embodiment is a 21st embodiment of the invention, that preferably depends on any of the 1st to 20th embodiments of the invention.

In the 21st embodiment, the wt-% is based on the weight of the particulated porous catalyst added in step b. of the 1st embodiment.

In a preferred embodiment of the method for providing a reactor at least partially filled with a reactant mass, during the step of opening the at least one outlet (step d. in the 1st embodiment), the amount of the particulated porous catalyst that exits the inner volume with the at least partial volume of the first mixture is less than 1000 ppm/s, preferably less than 100 ppm/s, more preferably less than 10 ppm/s, even more preferably less than 1 ppm/s, and further preferably less than 0.1 ppm/s. This preferred embodiment is a 22nd embodiment of the invention, that preferably depends on any of the 1st to 21st embodiments of the invention.

In a preferred embodiment of the method for providing a reactor at least partially filled with a reactant mass, the at least partial volume of the first mixture that exits the inner volume is subjected to at least one or all of the following steps:

> a. a liquid-solid separation step to remove at least 50 wt-%, preferably at least 75 wt-%, and more preferably at least 85 wt-% of the particulated porous catalyst that is carried out by the first mixture, where the wt-% are based on the total weight of the particulated porous catalyst that is carried out by the first mixture;
> b. feeding at least 40 vol-%, preferably at least 50 vol-%, and more preferably at least 60 vol% of the at least partial volume of the first mixture that exits the inner volume back into the inner volume.

This preferred embodiment is a 23rd embodiment of the invention, that preferably depends on any of the 1st to 22nd embodiments of the invention.

In an aspect of the 23rd embodiment, all possible combination of the features a. and b. are preferred aspects of the embodiment. These combinations are *e.g.,* a; b; a+b. In this aspect, for the combination a+b it is preferred to first perform feature a, then feature b.

A 24th embodiment of the invention is a reactor at least partially filled with a reactant mass obtainable according to a method for providing a reactor at least partially filled with a reactant mass, preferably the method according to any of the 1st to 23rd embodiments of the invention.

In a preferred embodiment of the reactor at least partially filled with a reactant mass, in the range of 50 vol-% to 95 vol-%, preferably in the range of 60 vol-% to 90 vol-%, and more preferably in the range of 70 vol-% to 85 vol-% of the inner volume of the reactor is filled with the reactant mass, where the vol% are based on a total volume of the inner volume. This preferred embodiment is a 25th embodiment of the invention, that preferably depends on the 24th embodiment of the invention.

In a preferred embodiment of the reactor at least partially filled with a reactant mass, the reactant mass comprises at least one or all of the following:

> a. in the range of 0.01 wt-% to 30 wt-%, preferably in the range of 0.05 wt-% to 25 wt-%, and more preferably in the range of 0.1 wt-% to 20 wt-% of the particulated porous catalyst;
> b. in the range of 30 wt-% to 99.9 wt-%, preferably in the range of 40 wt-% to 90 wt-%, and more preferably in the range of 50 wt-% to 80 wt-% of the first liquid.

This preferred embodiment is a 26th embodiment of the invention, that preferably depends on any of the 24th to 25th embodiments of the invention.

In an aspect of the 26th embodiment, all possible combination of the features a. and b. are preferred aspects of the embodiment. These combinations are *e.g.,* a; b; a+b.

In a preferred embodiment of the reactor at least partially filled with a reactant mass, the particles forming part of the particulated porous catalyst in the reactant mass has a $D_1$ in the range of 10 $\mu$m to 80 $\mu$m, preferably in the range of 15 $\mu$m to 60 $\mu$m, and more preferably in the range of 20 $\mu$m to 50 $\mu$m. This preferred embodiment is a 27th embodiment of the invention, that preferably depends on any of the 24th to 26th embodiments of the invention.

In a preferred embodiment of the 27th embodiment, the particles forming part of the particulated porous catalyst in the reactant mass has a $D_{90}$ in the range of 78 $\mu$m to 133 $\mu$m, preferably in the range of 88 $\mu$m to 123 $\mu$m, and more preferably in the range of 98 $\mu$m to 113 $\mu$m. In a preferred embodiment of the 27th embodiment, the particles forming part of the particulated porous catalyst in the reactant mass has a ratio $(D_1/D_{90})^{after}$ in the range of 0.28 to 0.6, preferably in the range of 0.34 to 0.50, and more preferably in the range of 0.38 to 0.46.

A 28th embodiment of the invention is a method for producing a product stream, comprising the steps of

> a. providing a reactor at least partially filled with a reactant mass obtainable according to the invention, preferably according to any of the 1st to 23rd, and 24th to 27th embodiments of the invention, wherein the reactant mass comprises the particulated porous catalyst;
> b. feeding a further liquid feed stream into an inner volume of the reactor containing the reactant mass, wherein
>
> > i. the further liquid feed stream comprises at least one liquid reactant,
> > ii. the further liquid feed stream and the reactant mass is at least partially mixed to obtain a further mixture;
>
> c. reacting the further mixture in the presence of the particulated porous catalyst to obtain a product stream, wherein the reaction is preferably performed at a temperature $T_2$.

In a preferred embodiment of the method for producing a product stream, the further mixture comprises the first liquid. This preferred embodiment is a 29th embodiment of the invention, that preferably depends on the 28th embodiment of the invention.

In an aspect of the 29th embodiment, it is preferred that the first liquid a.) forms part of the further liquid feed stream that is fed to the inner volume, b.) forms part of the reactant mass, or both a.) and b.).

In a preferred embodiment of the method for producing a product stream $T_2 > T_1$. This preferred embodiment is a 30th embodiment of the invention, that preferably depends on any of the 28th to 29th embodiments of the invention.

In a preferred embodiment of the method for producing a product stream, the further mixture is reacted at a temperature that is in the range of 50 °C to 110 °C, preferably in the range of 60 °C to 105 °C, and more preferably in the range of 70 °C to 98 °C. This preferred embodiment is a 31st embodiment of the invention, that preferably depends on any of the 28th to 30th embodiments of the invention.

In a preferred embodiment of the method for producing a product stream, the method further comprises the step of allowing at least one or all of the following to flow through the at least one liquid-solid separation means with a surface loading rate of $V_3$: the further mixture, the product stream, or both. This preferred embodiment is a 32nd embodiment of the invention, that preferably depends on any of the 28th to 31st embodiments of the invention.

In an aspect of the 32nd embodiment, it is preferred that the product stream makes up at least 10 vol-%, preferably at least 20 vol-%, and further preferably at least 30 vol-% of any liquid that flows through the at least one outlet.

In a preferred embodiment of the method for producing a product stream, $V_3 \leq V_2$, and preferably $V_1 < V_2$. This preferred embodiment is a 33rd embodiment of the invention, that preferably depends on any of the 28th to 32nd embodiments of the invention.

In a preferred embodiment of the method for producing a product stream, the at least one liquid reactant is at least one or all of the following: a $C_2$ - $C_8$ aldehyde, preferably an acrolein, more preferably methacrolein; an alcohol, more preferably methanol; water; a carboxylic acid, preferably methacrylic acid, a carboxylic acid salt, preferably sodium methacrylate; or a combination of at least two thereof. This preferred embodiment is a 34th embodiment of the invention, that preferably depends on any of the 28th to 33rd embodiments of the invention.

In a preferred embodiment of the method for producing a product stream, the further liquid feed stream comprises at least one or all of the following:

a. less than 0.1 wt-%, preferably less than 0.01 wt-%, and more preferably less than 0.001 wt-% of the particulated porous catalyst;
b. in the range of 30 wt-% to 99 wt-%, preferably in the range of 40 wt-% to 95 wt-%, and more preferably in the range of 50 wt-% to 90 wt-%, of the first liquid;
c. in the range of 1 wt-% to 30 wt-%, preferably in the range of 2 wt-% to 25 wt-%, and more preferably in the range of 5 wt-% to 20 wt-% of the at least one liquid reactant.

This preferred embodiment is a 35th embodiment of the invention, that preferably depends on any of the 28th to 34th embodiments of the invention.

In the 35th embodiment, the wt-% are based on the total weight of the further liquid feed stream. In an aspect of the 35th embodiment, all possible combination of the features a. to c. are preferred aspects of the embodiment. These combinations are *e.g.,* a; b; c; a+b; a+c; b+c; a+b+c.

In a preferred embodiment of the method for producing a product stream, a pressure in the reactor is in the range of 1 bar absolute to 15 bar absolute, preferably in the range of 1.5 bar absolute to 10 bar absolute, and more preferably in the range of 2 bar absolute to 6 bar absolute. This preferred embodiment is a 36th embodiment of the invention, that preferably depends on any of the 28th to 35th embodiments of the invention.

In a preferred embodiment of the method for producing a product stream, the product stream comprises an unsaturated alkyl ester, further preferably an alk-alkyl ester, even more preferably a meth-alkyl ester, and in particular preferred methyl methacrylate. This preferred embodiment is a 37th embodiment of the invention, that preferably depends on any of the 28th to 36th embodiments of the invention.

In a preferred embodiment of the method for producing a product stream, the product stream comprises at least 1 wt-%, more preferably at least 3 wt-%, and further preferably at least 10 wt-% of the unsaturated alkyl ester, where the wt-% is based on the total mass of the product stream. This preferred embodiment is a 38th embodiment of the invention, that preferably depends on any of the 28th to 37th embodiments of the invention.

In a preferred embodiment of the method for producing a product stream, the method further comprises the step of feeding a further gaseous feed stream into the reactant mass, wherein the further gaseous feed stream comprises at least one or all of the following:

a. at least 2 vol-%, preferably at least 4 vol-%, and more preferably at least 6 vol-% oxygen;
b. at least 50 vol-%, preferably at least 60 vol-%, and further preferably at least 70 vol-% $N_2$.

This preferred embodiment is a 39th embodiment of the invention, that preferably depends on any of the 28th to 38th embodiments of the invention.

In the 39th embodiment, the vol-% are based on a total volume of the further gaseous feed stream. In an aspect of the 39th embodiment, all possible combination of the features a. and b. are preferred aspects of the embodiment. These combinations are *e.g.,* a; b; a+b.

A 40th embodiment of the invention is a use of a reactor at least partially filled with a reactant mass according to the invention, preferably a reactor at least partially filled with a reactant mass obtainable according to any of the 1st to 23rd, and 24th to 27th embodiments of the invention, for producing an unsaturated alkyl ester, preferably an alk alkyl ester, more preferably a meth alkyl ester, and further preferably methyl methacrylate.

A 41st embodiment of the invention is an unsaturated alkyl ester obtainable by a method, according to the invention, for producing a product stream, preferably the method according to any of the 28th to 39th embodiments of the invention.

A 42nd embodiment of the invention is a use of an unsaturated alkyl ester obtainable by a method, according to the invention, for producing a product stream, preferably the method according to any of the 28th to 39th embodiments of the invention for producing a polymer.

A 43rd embodiment of the invention is a method for producing a polymer, preferably poly methyl methacrylate, comprising the steps

> a. providing a monomer obtainable by a method, according to the invention, for producing a product stream, preferably according to any of the 28th to 39th embodiments of the invention,
> b. polymerising the monomer to obtain the polymer.

A 44th embodiment of the invention is a polymer obtainable by a method, according to the invention, for producing a polymer, preferably the method according to the 43rd embodiment.

## DETAILED DESCRIPTION OF THE INVENTION

A *"pre-reactant mass"* should preferably be understood as referring to a liquid mixture that comprises at least one chemical substance, at least one chemical compound, or both. In one aspect of the invention, it is preferred that the least one chemical substance, the at least one chemical compound, or both, in the *"pre-reactant mass"* are used in a chemical reaction to obtain a product stream. In another aspect of the invention, it is preferred that a *"pre-reactant mass"* further comprises at least one solid, more preferably a solid catalyst, further preferably a particulated porous catalyst.

A *"reactant mass"* should preferably be understood as referring to a liquid mixture that comprises at least one chemical substance, at least one chemical compound, or both. In one aspect of the invention, it is preferred that the least one chemical substance, the at least one chemical compound, or both, in the *"reactant mass"* are used in a chemical reaction to obtain a product stream. In another aspect of the invention, it is preferred that a *"reactant mass "* further comprises at least one solid, more preferably a solid catalyst, further preferably a particulated porous catalyst.

A *"reactant mass"* is distinguished from a *"pre-reactant mass"* by at least one physical property of the liquid mixture being different between the *"reactant mass"* and the *"pre-reactant mass".* Examples of the at least one physical property include the temperature *(e.g.,* the *"reactant mass"* has a different temperature compared to the temperature of the *"pre-reactant mass");* the vol-% of at least one chemical substance, chemical composition or both, present in the liquid mixture; the wt-% of at least one chemical substance, chemical composition or both, present in the liquid mixture; the particle size distribution of a solid present in the liquid mixture *(e.g.,* the *"reactant mass"* has a smaller number of particles, forming part of a particulated porous catalyst, with a size below 20 $\mu$m, as compared the number of catalyst particles with a size below 20 $\mu$m in the *"pre-reactant mass"*); the density of the liquid mixture; the pH of the liquid mixture; the conductivity of the liquid mixture. In an aspect of the invention, it is preferred to use at least a partial volume of the *"reactant mass"* in a method for producing a product stream, *e.g.,* the "*reactant mass*" is used as part of the feed stream and/or starting material in a method for producing a product stream, wherein the product stream comprises, *e.g.*, methyl methacrylate.

A reactor suitable for the present invention is well-known to the skilled person, and is commercially available from *e.g.*, Barlage GmbH (Germany) and Paul Mueller (USA). Suitable reactors include reactors used for the production of methacrolein, methacrylic acid, and methyl methacrylate.

In an aspect of the invention, it is preferred that the inner volume of the reactor suitable for the invention has a first reaction zone and a further zone. In this aspect, it is preferred that the first zone and the further zone are at least partially separated by a physical means, such as an internal reactor wall. In this aspect, it is preferred that the first zone and the further zone are in fluid communication with each other.

A "*liquid-solid separation means"* should preferably be understood as a means that is adapted and arranged to at least partially separate a solid from a liquid, *e.g.,* a filter, a clarifier (such as a lamella clarifier), or a settling tank. A liquid-solid separation means that is suitable for the present invention is well-known to the skilled person, and is commercially available from *e.g.*, Haecker Maschinen GmbH (Germany) and HST Systemtechnik GmbH & Co. KG (Germany).

A "*surface loading rate*" of a liquid-solid separation means should preferably be understood as the volumetric flow rate of a liquid flowing through the liquid-solid separation means, divided by the total surface area of the one or more physical surfaces of the liquid-solid separation means used to perform the liquid-solid separation. For a lamella clarifier, each plate (or lamella) in the lamella clarifier is projected onto the horizontal plane, with the area of the projection measured. The total surface area of the lamella clarifier is obtained by summing the projected surface areas of the individual plates (or lamellae). An increase in the "*surface loading rate*" preferably increases the liquid-solid separation of the liquid-solid separation means.

A "*filling density*" of the particulated porous catalyst should preferably be understood as the density of the particulated porous catalyst when the open pores of the particles forming the particulated porous catalyst are at least partially filled with a liquid, preferably the first liquid, an even-further liquid, or a combination thereof.

The "*envelope density*" of the particulated porous catalyst should preferably be understood as the mass of the particles forming the particulated porous catalyst, divided by the volume of said particles, where the volume of the particle includes the open and closed pores present in the particles.

The "*true density*" of the particulated porous catalyst should preferably be understood as the mass of the particles forming the particulated porous catalyst, divided by the volume of said particles, where the volume of a particles excludes the open and closed pores present in the particles. For example, if the particulated porous catalyst consists of graphite, the density of the particulated porous catalyst is equal to the density of graphite (2.3 g/cm$^3$).

In an aspect of the invention, it is preferred that the first liquid has a density in the range of 700 kg/m$^3$ to 900 kg/m$^3$ at 80 °C. In an aspect of the invention, it is preferred that the first liquid has a viscosity in the range of 0.24 mPa·s to 0.39 mPa·s at 80 °C.

The "$D_1$" and "$D_{90}$" values should preferably be understood as characterising the particle size distribution of the particles making up the particulated porous catalyst, *i.e.,* 1 % of the particles have a diameter less than the value of Di, while 90% of the particles have a diameter less than the value of $D_{90}$.

A "*reactant*" should preferably be understood to refer to a chemical substance and/or a chemical compound that is used to obtain a product stream. For example, methanol is a reactant that is used to obtain a product stream that comprise methyl methacrylate.

A "*particulated porous catalyst*" should preferably be understood as a catalyst that is in the form of a plurality of particles, wherein the particles comprise spaces, voids, hollows, pores, or a combination of at least two thereof. Suitable catalysts are well-known to the skilled person.

The invention is now illustrated by non-limiting examples and exemplifying embodiments.

**FIGURES**

List of figures

The figures serve to exemplify the present invention, and should not be viewed as limiting the invention. The figures are not drawn to scale.

Fig. 1:  schematic illustration of the method for providing a reactor that is at least partially filled with a reactant mass.
Fig. 2:  schematic illustration of the flow through a liquid separation means.
Fig. 3:  flow diagram showing the steps of the method for providing a reactor hat is at least partially filled with a reactant mass.
Fig. 4:  flow diagram showing the steps of the method for producing a product stream.
Fig. 5:  graphs showing the change in the $D_i$ values for the particle size of the particulated porous catalyst as a function of time (normalised to the clarification time).
Fig. 6:  graphs showing the change in the ratios $D_x/D_y$ for the particle size of the particulated porous catalyst as a function of time (normalised to the clarification time).
Fig. 7:  drawing illustrating how the projected surface area of an individual lamella, forming part of a lamella clarifier, is calculated

Description of figures

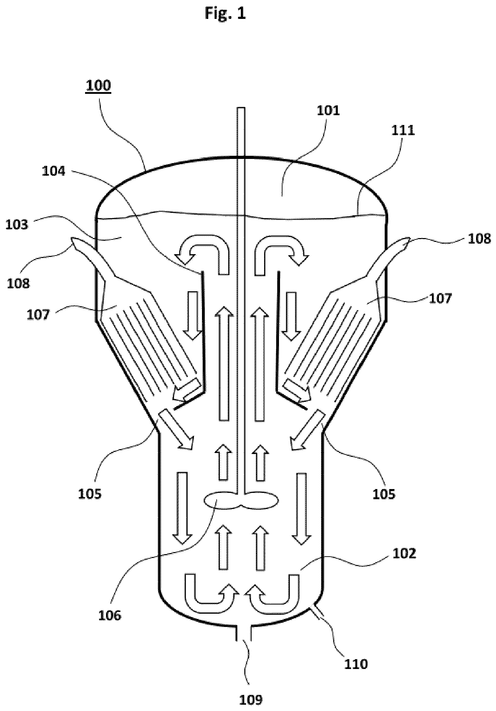

Fig. 1 is a schematic illustration of the method for providing a reactor that is at least partially filled with a reactant mass. A reactor 100 with an inner volume 101 is provided. The inner volume 101 is divided into a first zone 102 and a further zone 103, wherein the first zone 102 and the further zone 103 are partially separated from each other by an internal wall 104. A liquid can circulate in the inner volume 101 between the first zone 102 and the further zone 103 by flowing over the internal wall 104 into the further zone 103 and through internal openings 105 into the first zone 102, as shown by the arrows in Fig. 1. Alternatively, the liquid can circulate in the inner volume 101 between the first zone 102 and the further zone 103 by flowing through the internal openings 105 and over the internal wall 104. The reactor 100 also comprises an agitation means 106 in the form of a stirrer, which is located in the first zone 102. The reactor 100 further comprises two liquid-solid separation means 107 in the form of lamella clarifiers, which are located in the further zone 103. Each liquid-solid separation means 107 has an outlet 108 for allowing a liquid that has flowed through the liquid-solid separation means 107 to exit the reactor 100. Initially the outlets 108 are closed. The reactor further comprises an inlet for a liquid feed stream 109 and an inlet for a gaseous feed stream 110. A liquid in the inner volume 101 can also be agitated by feeding a liquid feed stream, a gaseous feed stream, or both, into the inner volume 101.

After providing the reactor 100 with the outlets 108 closed, the inner volume 101 is partially filled with a first mixture that comprises a particulated porous catalyst and a first liquid. The volume of the first mixture defines a pre-reactant mass. The level 111 of the first mixture is shown in Fig. 1. The first mixture is subsequently agitated using the agitation means 106, thereby allowing the first mixture to circulate through the inner volume 101 as shown in Fig. 1. As a result of the opening 108 being closed, a surface loading rate $V_1$ of the first mixture through the liquid-solid separation means 107 is $V_1 = 0$ m$^3$/(h·m$^2$). After the circulation of the first mixture through the inner volume 101 has started, the outlets 108 are opened, thereby allowing a partial volume of the first mixture to flow through the liquid-solid separation means 107 and to exit the reactor 100 via outlets 108. Once the outlets 108 are opened, the surface loading rate of the first mixture through the two liquid-solid separation means is $V_2$, where $V_2 > V_1$, and $V_d$ 0. The first mixture is allowed to exit the reactor 100 until the $D_1$ value of the particulated porous catalyst in the inner volume has increased to a desired value. The first mixture remaining in the inner volume 101 defines the reactant mass. In performing the method for providing the reactor at least partially filled with the reactant mass, less than 5 vol-% of first liquid is converted to a different chemical substance or chemical compound by means of a chemical process. The vol-% is based on the total volume of the first liquid added to the reactor.

Additional first liquid may be added continually and/or batch-wise to the reactor 100 after the outlets 108 have been opened. This additional first liquid is mixed with the first mixture present in the inner volume 101, thereby forming part of the first mixture. At least a partial volume of the additional first liquid (forming part of the first mixture) may also exit the reactor via the outlets 108. In other words, the first mixture in the inner volume 101 is at least partially replenished.

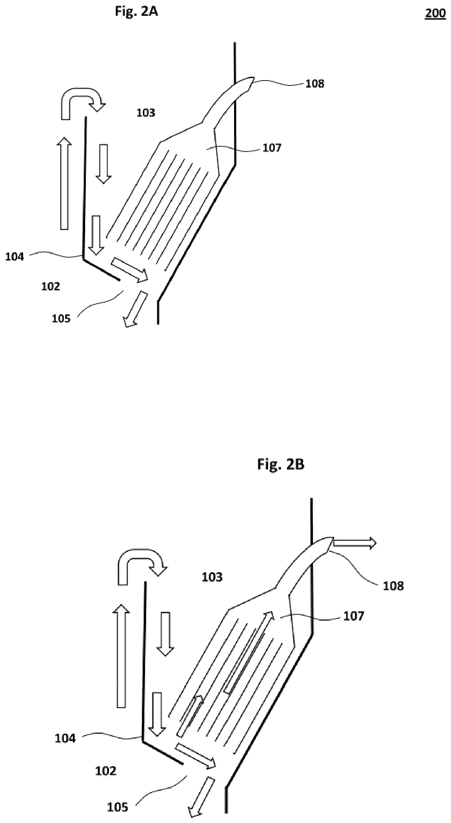

Fig. 2 is a cut-out and an enlargement of a liquid-solid separation means 107 shown in Fig. 1. Fig. 2A shows the flow of the first mixture in the reactor when the outlets 108 are closed. While some of the first mixture will fill the liquid-solid separation means 107, most of the first mixture circulates past the liquid-solid separation means 107, and between the first zone 102 and the further zone 103. This flow is indicated by the arrows in Fig. 2A. Fig. 2B shows the flow of the first mixture in the reactor when the outlets 108 are opened. While some of the first mixture flows past the liquid-solid separation means 107, a partial volume of the first mixture flows through the liquid-solid separation means 107 and exits the reactor through the outlets 108. This flow is indicated by the arrows in Fig. 2B. When flowing through the liquid-solid separation means 107, larger particles that form part of the particulated porous catalyst (which in turn forms part of the first mixture) will generally be settled in the liquid-solid separation means 107. As a result of gravity, these settled particles will move towards the opening 105, and thus remain in the inner volume of the reactor. However, smaller particles that form part of the particulated porous catalyst will generally be carried out by the partial volume of the first mixture that exits through the outlet 108. A reactor that is at least partially filled with the reactant mass is obtained once a desired number of these smaller particles have been removed from the reactor. The reactant mass therefore comprises the first liquid and the particulated porous catalyst with a $D_1$ value that a larger than the $D_1$ value of the particulated porous catalyst in the pre-reactant mass.

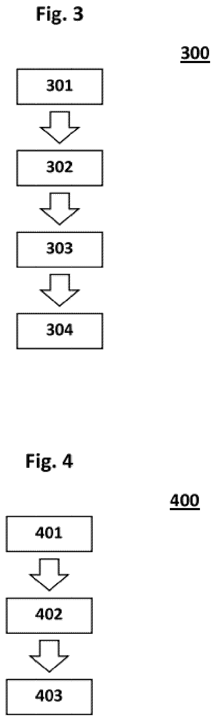

Fig. 3 is a flow diagram showing the steps of the method for providing a reactor that is at least partially filled with a reactant mass. In step 301 a reactor that has an inner volume is provided. The reactor has an agitation means and two liquid-solid separation means that are both located in the inner volume. The two liquid-solid separation means have outlets that are closed. In step 302 the inner volume is partially filled with a first mixture that comprises a particulated porous catalyst and a first liquid. The volume of the first mixture defines a pre-reactant mass. In step 303, the first mixture in the inner volume is agitated using the agitation means, thereby obtaining a circulation of the first mixture in the inner volume, with a surface loading rate of the first mixture through the at least one liquid-solid separation means being $V_1$ = 0 m$^3$/(h·m$^2$). In step 304, the outlets of the liquid-solid separation means are opened to allow a partial volume of the first mixture to exit the inner volume through the outlets, wherein the surface loading rate of the first mixture through the two liquid-solid separation means is $V_2 > V_1$. First liquid is also continually added to the inner volume and mixed with the remaining first mixture in the inner volume of the reactor. This is done to at least partially replenish the first mixture that has exited the reactor. The first mixture is allowed to exit the reactor until the $D_1$ value of the particulated porous catalyst in the inner volume of the reactor has increased to a desired value. The reactant mass is defined by the volume of the first mixture remaining in the inner volume of the reactor after the desired $D_1$ value has been obtained.

Fig. 4 is a flow diagram showing the steps of the method for producing a product stream. In step 401, a reactor at least partially filled with a reactant mass is provided. This reactor is provided by performing a method, according to the invention, for providing a reactor that is at least partially filled with a reactant mass. The reactant mass is defined by the volume of the first mixture, which comprises the particulated porous catalyst and the first liquid. In step 402 a further liquid feed stream is fed into the inner volume of the reactor containing the reactant mass, wherein the further liquid feed stream comprises a further liquid. The further liquid feed stream and the reactant mass are mixed to obtain a further mixture. The first liquid and the further liquid are both reactants used to obtain the product stream. In step 403, the further mixture is reacted in the presence of the particulated porous catalyst to obtain a product stream.

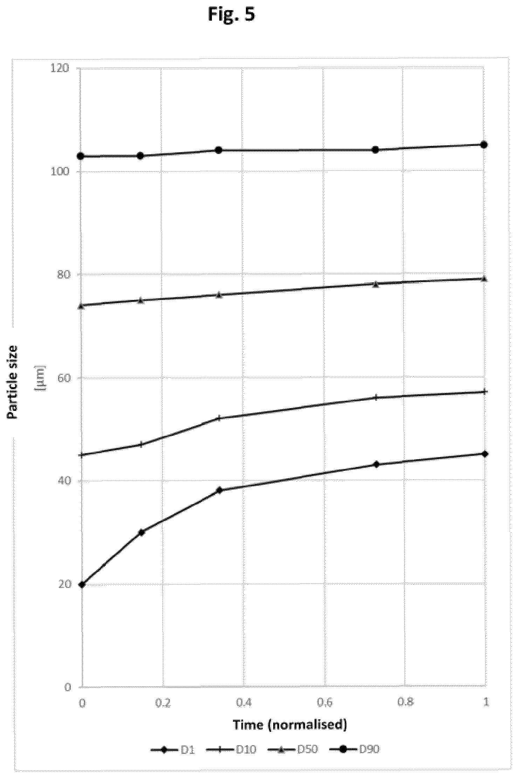

Fig. 5 shows experimental measurements of the change in the $D_i$ values for the particle size of the particulated porous catalyst as a function of time. The particulated porous catalyst is located in the inner volume of the reactor and forms part of the first mixture. The $D_i$ values shown in Fig. 5 are measured at evenly-spaced time intervals $t_0, t_1, t_2, ...$ $t_j$, where $t_0$ is the measurement made prior to "*at least partially opening the at least one outlet to allow at least a partial volume of the first mixture to exit the inner volume through the at least one outlet*". The x-axis has been normalised with respect to a clarification time. The clarification time is defined as the time required for the values of $D_1$, $D_{10}$, $D_{50}$, and $D_{90}$ to change by less than 5 % between two subsequent measurements. More specifically, the time $t_j$ where $\Delta D_1 < 5\%$, $\Delta D_{10} < 5\%$, $\Delta D_{50} < 5\%$, and $\Delta D_{90} < 5\%$, with

$$\Delta D_i = [D_i(t_j) - D_i(t_{j-1})] / D_i(t_{j-1})] * 100 \%,$$

is defined as the clarification time.

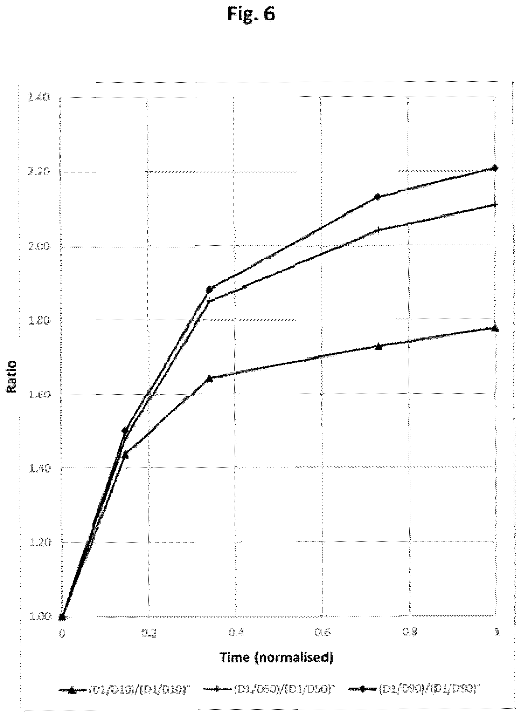

Fig. 6 shows experimental measurements of the change in the ratios Dx/Dy for the particle size of the particulated porous catalyst as a function of time. The particulated porous catalyst is located in the inner volume of the reactor and forms part of the first mixture. The $D_i$ values shown in Fig. 6 are measured at evenly-spaced time intervals $t_0, t_1, t_2, ...$ $t_j$, where $t_0$ is the measurement made prior to "*at least partially opening the at least one outlet to allow at least a partial volume of the first mixture to exit the inner volume through the at least one outlet*". Similar to Fig. 5, the x-axis has been normalised with respect to the clarification time.

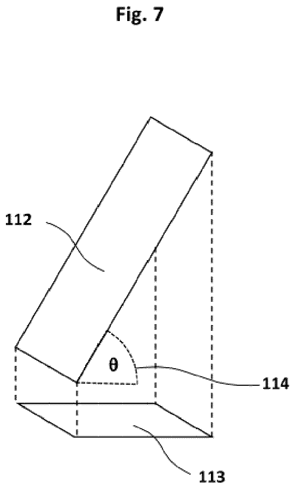

Fig. 7 illustrates how the projected surface area of an individual lamella, forming part of a lamella clarifier, is calculated. The lamella 112 is inclined at an angle $\theta$ (114) with respect to the horizontal plane. The projected surface area $A_{proj}$ (113) of the lamella 112 is calculated as follows:

$$A_{proj} = A_{lamella} \cdot \cos\theta,$$

where $A_{lamella}$ is the surface area of the lamella 112. The total surface area of the lamella clarifier is obtained by summing the projected surfaces areas of the individual lamellae.

**TEST METHODS**

The test methods which follow were utilized within the context of the invention. Unless stated otherwise, the measurements were conducted at an ambient temperature of 23 °C and an ambient air pressure of 100 kPa (0.986 atm).

Surface loading rate

A lamella clarifier comprises a number of surfaces (or lamellae) arranged at an incline with respect to the horizontal plane. The lamellae are the physical surfaces used for performing the liquid-solid separation. To calculate the surface loading rate of the lamella clarifier, the volumetric flow rate of a liquid flowing through the lamella clarifier is divided by the total surface area of the lamella clarifier. The total surface area is obtained by summing the projected surface areas of the individual lamellae. The calculation of the projected surface area of an individual lamella is shown in Fig. 7.

Filling density

The filling density is determined as follows:

i. The total pore volume per unit weight, $\Gamma_{PORES}$, of the particulated porous catalyst is determined using the standard ASTM D4284-12(2017)e1. The total pore volume per unit weight has units of [volume]/[mass], *e.g.*, cm$^3$/g.

ii. A vessel, suitable for measuring volume, is filled with 2 litres of the first liquid. A 1 kg sample of the particulated porous catalyst is added to the measuring vessel. The measuring vessel should be chosen such that the particulated porous catalyst is completely covered by the first liquid. The mixture of the first liquid and the particulated porous catalyst is left to stand for 24 hours. The volume of the mixture of the first liquid and the particulated porous catalyst is then measured after 24 hours.

iii. The volume of the sample of the particulated porous catalyst, excluding the volume of the open pores in the particulated porous catalyst, is calculated using the following equation:

$$V_{\text{PPC, NO PORES}} = V_{\text{TOT, MIXTURE}} - V_{\text{FL}},$$

where $V_{\text{PPC, NO PORES}}$ is the volume of the particulated porous catalyst, excluding the volume of the open pores in the particulated porous catalyst, $V_{\text{TOT, MIXTURE}}$ is the volume of the mixture of the first liquid and the particulated porous catalyst, and $V_{\text{FL}}$ (= 2 litre) is the volume of the first liquid.

iv. The volume of the sample of the particulated porous catalyst, including open pores, $V_{\text{PPC, TOTAL}}$, is calculated using the following equation.

$$V_{\text{PPC, TOTAL}} = V_{\text{PPC, NO PORES}} + \Gamma_{\text{PORES}} \cdot M_{\text{PPC}},$$

where $M_{\text{PPC}}$ is the mass (1 kg) of the sample of the particulated porous catalyst.

v. The filling density of the particulated porous catalyst, $\rho_{\text{FILLING}}$, is determined by the following equation:

$$\rho_{\text{FILLING}} = (M_{\text{PPC}} + \Gamma_{\text{PORES}} \cdot M_{\text{PPC}} \cdot \rho_{\text{FL}}) / V_{\text{PPC, TOTAL}},$$

where $\rho_{\text{FL}}$ is the density of the first liquid.

Particle size

The particle size of the particles forming part of the particulated porous catalyst are measured using the Camsizre X2 particle size and shape analyser, commercially obtainable from Microtrac Retsch GmbH (Germany). From these measurements the $D_i$ (e.g., $D_1$) values for the particle size are obtained.

True density

True density is measured using an AccuPyc II 1340 Gas Displacement Pycnometry System commercially available from Micromeritics Instrument Corporation (USA)

Envelope density

Envelope density is measured using the GeoPyc 1360 Envelope Density Analyzer commercially available from Micromeritics Instrument Corporation (USA).

Dynamic viscosity

The kinematic viscosity of the first liquid is first measured using an Ubbelohde viscosimeter, according to the standard DIN 51562-1:1999. The dynamic viscosity is then calculated using the following equation:

$$\mu = \nu \rho,$$

where $\mu$ is the dynamic viscosity, $\nu$ is the kinematic viscosity, and p is the density of the first liquid.

Conversion of first liquid

The conversion of the first liquid to a different chemical substance or chemical compound is determined by measing the composition of the first mixture in the inner volume of the reactor. The concentration of the chemical components that make up the first mixture, with the exception water, is measured using gas chromatography. The concentration of water is measured using Karl Fischer titration.

The composition of the first mixture is measured prior to *"at least partially opening the at least one outlet to allow at least a partial volume of the first mixture to exit the inner volume through the at least one outlet"*, thereby obtaining the concentration $C_1$ of the first liquid. The composition of the first mixture is again measured after 20 vol-% of the first mixture has exited the reactor after the opening of the at least one outlet, thereby obtaining the concentration $C_2$ of the first liquid (the vol-% exiting the reactor is based on the volume of the first mixture prior to the opening of the at least one outlet). The conversion $\Delta C$ of the first liquid is determined as follows:

$$\Delta C = (C_2 - C_1) / C_1 \cdot 100\ \%.$$

Chemical composition

The chemical composition of a gaseous feed stream (*e.g.*, the first gaseous feed stream) is determined by gas chromatography. The methods and devices for making these measurements are well known to one skilled in the art.

**EXAMPLES**

The invention is illustrated further by way of examples. The invention is not restricted to the examples.

The particulated porous catalyst

The following particulated porous catalyst is used for the examples and the comparative examples described below: a particulated porous catalyst with a 0.85 wt-% gold on a $SiO_2$-$Al_2O_3$-MgO support. The particulated porous catalyst has a $D_1 = 20\ \mu m$. The $D_{10}$, $D_{50}$, and $D_{90}$ values are shown in Fig. 5. The filling density of the particulated porous catalyst is 2.1 g/cm$^3$. The catalyst and support is prepared as described below:

*Preparation of the $SiO_2$-$Al_2O_3$-MgO support:*

The support was obtained according to the following steps:

i. 21.36 kg of $Mg(NO_3)_2$*$6H_2O$ and 31.21 kg of $Al(NO_3)_3$*$9H_2O$ were placed together in an enamel-lined vessel and dissolved in 41.85 kg of deionised water by stirring the aforementioned components. The stirring was done using an impeller stirrer. Once dissolution was completed, 1.57 kg, 60% $HNO_3$ was added to the enamel-lined vessel, while stirring the mixture in said vessel.

ii. 166.67 kg of silica sol (30 wt-% $SiO_2$, mean particle size of 15 nm), commercially available from Chemiewerk Bad Köstritz GmbH (Germany) under the product name Köstrosol 1530AS, was placed in an enamel-lined reactor, and cooled to 15 °C under stirring with an impeller stirrer. 2.57 kg, 60% $HNO_3$ was slowly added to the silica sol while slowly stirring the silica sol.

iii. The contents of the enamel-lined vessel (obtained in step i.) was added to the enamel-lined reactor, *i.e.,* to the contents of the reactor (obtained in step ii.), thereby obtaining a combined mixture. The combined mixture was heated to 50 °C within 30 min and kept at this temperature for 4 hours, wherein the combined mixture gelled.

iv. Once gelled, the combined mixture was continuously pumped as a suspension into a spray dryer and spray dried at 130 °C to obtain a dried product. The dried product was subsequently calcined in a rotary kiln, wherein the residence time in the hot zone (600 °C) was 45 minutes. The residence time and throughput time of the calcination in the rotary kiln was regulated by the inclination of the rotary kiln (about 1 to 2° inclination). The dried product was passed through the rotary kiln a second time, using the same residence time and temperature settings in the hot zone. After the second calcination, 55 kg of a white, flowable, powdery carrier material was obtained. The nitrate content in the carrier material was less than 1000 ppm. The mass yield was about 95%. Furthermore, the carrier material was spherical, to a first approximation, with an average particle size distribution of 60 $\mu m$.

v. The carrier material was then sieved using several passes and different sieve sizes, wherein particles with sizes above 120 $\mu m$ and below 15 $\mu m$ were removed. Nevertheless, 3.6 vol-% of fine particles remained, *i.e.,* particles with a size between 1 and 15 $\mu m$. The yield of after sieving was 70 %, and the average particle size distribution of the carrier material, after sieving, was 68 $\mu m$.

*Preparation of the catalyst:*

The catalyst was obtained according to the following steps:

i. 15 kg of the carrier material was suspended in 50 kg of deionised water, in an enamel-lined reactor, with stirring using an impeller stirrer. The resulting mixture was heated to 90 °C and aged for 30 minutes after reaching 90 °C.

ii. A solution of 852.9 g $Co(NO_3)_2*6H_2O$ in 7.5 kg deionised water was added over 10 minutes to the contents of the reactor (obtained in step i.) and aged for 30 minutes.

iii. Subsequently, 3.7 litres of a 1 molar NaOH solution was added over 10 minutes to the contents of the reactor (obtained in step ii.).

iv. Subsequently, a solution of 376.8 g gold acid (41% gold content) in 7.5 kg deionised water was added to the contents of the reactor (obtained in step iii.) and aged for 30 minutes. The resulting suspension was cooled to 40 °C and filtered using a centrifuge. The filter cake (*i.e.*, the catalyst) was washed with deionised water in the centrifuge until the filtrate became clear and had a conductivity below 100 μS/cm.

v. The wet catalyst was dried at 105 °C until the residual moisture was below 5%. The dried catalyst was calcined in a rotary kiln, wherein the residence time in the hot zone (450 °C) was 45 minutes. The mass yield was 99%. The catalyst obtained was a spherical, egg-shell catalyst, with a fines content of 3.5 vol% (particles with a size between 1 and 15 μm). The gold content catalyst was 0.85 wt-%.

Comparative example 1

A reactor as shown in Fig. 1 is provided, *i.e.*, the reactor comprises an inner volume in which a stirrer (an agitation means) and two lamella clarifiers (liquid-solid separation means) are located. Each lamella clarifier has an outlet.

80 % of the inner volume is filled with a liquid feed stream that comprises methanol (60 wt-%), water (5 wt-%), methacrylic acid and its sodium salt in a ratio that leads to the liquid feed stream having a pH of 7 ± 0.25 (the combined weight of the methacrylic acid and its sodium salt is 1.5 wt-%), and methyl methacrylate (33 wt-%). The wt-% are based on the total weight of the liquid feed stream. The total weight of the liquid feed stream is 130 kg. 13 kg of particulated porous catalyst is added to the liquid feed stream in the inner volume. The methanol, water, methacrylic acid, methyl methacrylate, and particulated porous catalyst are mixed to obtain a further mixture. The pressure in the inner volume set to 5 bar absolute by feeding in nitrogen into the reactor, while the liquid feed stream is heated to an operating temperature of 80 °C. Once the operating temperature has been reached, methacrolein is fed into the reactor at a rate of 140 mol per hour, along with methanol. The molar ratio of the methanol to methacrolein is 4. During operation of the reactor, air is also fed into the reactor at a rate of 1 kg/h.

The operation of the reactor under the above described conditions leads to the production of methyl methacrylate in the reactor. A partial volume of the further mixture is allowed to exit the reactor via the outlets of the lamella clarifiers, *i.e.,* said partial volume flows through the lamella clarifiers. The further mixture that exits the reactor is subsequently filtered.

Example 1

A reactor as shown in Fig. 1 is provided, *i.e.*, the reactor comprises an inner volume in which a stirrer (an agitation means) and two lamella clarifiers (liquid-solid separation means) are located. Each lamella clarifier has an outlet which is closed.

80 % of the inner volume is filled with a first mixture that comprises 120 kg methanol (a first liquid) and 13 kg of the particulated porous catalyst. The open pores of the particulated porous catalyst are at least partially filled with methanol. The volume of the first mixture defines the pre-reactant mass. The filling density of the particulated porous catalyst is 2.1 g/cm$^3$, while the density of methanol is 0.77 cm$^3$. The temperature and pressure in the inner volume of the reactor is 45 °C and 2 bar absolute, respectively. Any methanol evaporating in the reactor is condensed and returned to the inner volume of the reactor.

The first mixture is agitated for 1 hour, during which the surface loading rate of the first mixture through the lamella clarifiers is $V_1 = 0$ m$^3$/(h·m$^2$). After 1 hour, the outlets of the lamella clarifiers are opened, thereby allowing the first mixture to exit the reactor via the lamella clarifiers and their outlets. The surface loading rate of the first mixture through the lamella clarifiers is $V_2 = 0.8$ m$^3$/(h·m$^2$). While the outlets are opened, methanol is continuously fed into the reactor, keeping the inner volume of the reactor 80 % filled. The newly fed methanol also forms part of the first mixture. The process is continued until the clarification time is reached. The first mixture remaining in the reactor defines the reactant mass.

Fig. 5 shows the change in the $D_i$ of the particulated porous catalyst as a function of time, with the time normalised to the clarification time. The values at x = 0 (normalised time = 0) corresponds to the $D_i$ values prior to the opening of the outlets of the lamella clarifiers. Fig.5 shows a significant increase in the $D_1$ value, especially between the times x = 0 and x = 0.4, indicating that the smallest particles of the particulated porous catalyst are being removed from the reactor. By contrast, the values of $D_{50}$ and $D_{90}$ show only a moderate change, indicating that the larger particles of the particulated porous catalyst remain in the reactor.

Fig. 6 shows the change in the ratios $D_x/D_y$ as a function of time, with the time normalised to the clarification time. The ratio $(D_x/D_y)^0$ is the ratio prior to the opening of the outlets of the lamella clarifiers. The values at x = 0 (normalised time = 0) correspond to the ratios prior to the opening of the outlets of the lamella clarifiers, *i.e.,* $(D_x/D_y)^0$. Fig. 6 also shows that the smallest particles are removed from the reactor, while the larger particles remain in the reactor.

A liquid feed stream (the further liquid feed stream) that comprises water (<1 wt-%), methanol (50 wt-%) and methyl methacrylate (8 wt-%) is added to inner volume of the reactor and mixed with the reactant mass to obtain a further mixture. The wt-% are based on the total weight of the liquid feed stream. The liquid components in the further mixture has a weight of 130 kg, with the further mixture comprising methanol (60 wt-%), water (5 wt-%), methacrylic acid and its sodium salt in a ratio that leads to the liquid feed stream having a pH of 7 $\pm$ 0.25 (the combined weight of the methacrylic acid and its sodium salt is 1.5 wt-%), and methyl methacrylate (33 wt-%). The wt-% are based on the total weight of the liquid components present in the further mixture. The particulated porous catalyst in the further mixture has a weight of 13 kg.

The pressure in the inner volume set to 5 bar absolute by feeding in nitrogen into the reactor, while the liquid feed stream is heated to an operating temperature of 80 °C. Once the operating temperature has been reached, methacrolein is fed into the reactor at a rate of 130 mol per hour, along with methanol. The molar ratio of the methanol to methacrolein is 4. During operation of the reactor, air is also fed into the inner volume of the reactor with a flow that causes the residual oxygen content in a vent system of the reactor to be 4 vol.-%

The operation of the reactor under the above described conditions leads to the production of methyl methacrylate in the reactor, *i.e.,* a product stream comprising methyl methacrylate is obtained. A partial volume of the product stream is allowed to exit the reactor via the outlets of the lamella clarifiers, *i.e.,* said partial volume flows through the lamella clarifiers. The product stream that exits the reactor is subsequently filtered.

Table 1 shows the technical effects of using a reactant mass for the production of methyl methacrylate (Example 1) vs not using a reactant mass for the production of methyl methacrylate (Comparative example *1). I.e.,* the particulated porous catalyst in Comparative example 1 is not subjected to a process of increasing the value of $D_1$, while the $D_1$ value in Example 1 is increased prior to performing the method for obtaining methyl methacrylate.

*Table 1*

| Technical effect | Example 1 | Comparative example 1 |
|---|---|---|
| $D_1$ of particulated porous catalyst [$\mu$m] | 40 | 17 |
| Increase in pressure drop over filter | Not observed (< 1 mbar) | 30 mbar |
| Time until first filter maintenance after reaction start | >1000h | <100h |
| Amount of catalyst present in product stream | 12 ppm | 135 ppm |
| Product yield | Higher | Lower |
| Catalyst loss | Low | High |

The technical effects in Table 1 are as follows:

- $D_1$ of particulated porous catalyst: the $D_1$ of particulated porous catalyst present in the reactor during the production of methyl methacrylate.

- Increase in pressure drop over filter: the pressure measured between the entrance and exit of the filter, used to filter the product stream, decreases. This decrease is defined as the pressure drop. A clean filter has a certain pressure drop. When the pressure drop increases with time and usage of the filter, this indicates that the filter is being clogged, which in turn requires the filter to be either exchanged or cleaned.

- Time until first filter maintenance after reaction start: after the production of the product stream has started in the reactor, the up time of the reactor until maintenance of the filter is required for the first time.

- Amount of catalyst present in product stream: the amount of catalyst present in product stream exiting the reactor.

- Product yield: the amount of methyl methacrylate obtained from the reaction.

- Catalyst loss: used catalyst is recycled to produce a new catalyst. The smallest particles of the particulated porous catalyst is generally lost during the production of the product stream. It is thus desired to reduce the loss of these smallest particles.

**REFERENCE LIST**

| | |
|---|---|
| 100 | Reactor |
| 101 | Inner volume |
| 102 | First zone |
| 103 | Further zone |
| 104 | Internal wall |
| 105 | Internal opening |
| 106 | Agitation means |
| 107 | Liquid-solid separation means |
| 108 | Outlet |
| 109 | Inlet for liquid feed stream |
| 110 | Inlet for gaseous feed stream |
| 111 | Level of first mixture |
| 112 | Lamella |
| 113 | Projected surface area |
| 114 | Inclination angle |

**Claims**

1. A method for providing a reactor at least partially filled with a reactant mass, comprising the steps of

   a. providing the reactor that has an inner volume, wherein the reactor comprises

      i. at least one agitation means;
      ii. at least one liquid-solid separation means, wherein the at least one liquid-solid separation means

         A. comprises at least one outlet that is at least partially closed, and
         B. is located in the inner volume;

   b. at least partially filling the inner volume with a first mixture, wherein

      i. the volume of the first mixture defines a pre-reactant mass, and
      ii. the first mixture comprises

         A. a particulated porous catalyst, and
         B. a first liquid;

   c. agitating the first mixture in the inner volume with the at least one agitation means,

      i. thereby obtaining a circulation of the first mixture in the inner volume;

   d. at least partially opening the at least one outlet to allow at least a partial volume of the first mixture to exit the inner volume through the at least one outlet,

      i. thereby obtaining the reactant mass, wherein the reactant mass is defined as the volume of the first mixture remaining in the inner volume.

2. The method according to claim 1, wherein less than 10 vol-% of the first liquid in the inner volume is converted to a different chemical substance, chemical composition, or both, by a chemical reaction, where the vol-% is based on a total volume of the first liquid in the inner volume.

3. The method according to any of the preceding claims, wherein a filling density of the particulated porous catalyst is at least 1.2 times higher than a density of the first liquid in the inner volume.

4. The method according to any of the preceding claims, wherein the particulated porous catalyst has at least one or all of the following properties:

   a. the filling density of the particulated porous catalyst is in the range of 1 g/cm$^3$ to 3.6 g/cm$^3$;
   b. the density of the first liquid in the inner volume is in the range of 0.3 g/cm$^3$ to 1.9 g/cm$^3$.

5. The method according to any of the preceding claims, wherein the first liquid in the inner volume has a dynamic viscosity in the range of 0.01 mPa·s to 2.1 mPa·s.

6. The method according to any of the preceding claims, wherein the at least one liquid-solid separation means is a lamella clarifier.

7. The method according to any of the preceding claims, wherein at least one or all of the following applies:

   a. during agitation of the first mixture in the inner volume (step c.), the surface loading rate of the first mixture through the at least one liquid-solid separation means, $V_1$, is less than 0.05 m$^3$/(h·m$^2$);
   b. after opening of the at least one outlet to allow the at least a partial volume of the first mixture to exit the inner volume through the at least one outlet (step d.), the surface loading rate of the first mixture through the at least one liquid-solid separation means, $V_2$, is in the range of 0.2 m$^3$/(h·m$^2$) to 2 m$^3$/(h·m$^2$).

8. The method according to any of the preceding claims, wherein a ratio $D_1/D_{90}$ of the particulated porous catalyst in the inner volume is increased by a factor of at least 1.2.

9. A reactor at least partially filled with a reactant mass obtainable according to any of the preceding claims.

10. A method for producing a product stream, comprising the steps of

    a. providing a reactor at least partially filled with a reactant mass obtainable according to any of the preceding claims 1 to 8, wherein the reactant mass comprises the particulated porous catalyst;
    b. feeding a further liquid feed stream into an inner volume of the reactor containing the reactant mass, wherein

       i. the further liquid feed stream comprises at least one liquid reactant,
       ii. the further liquid feed stream and the reactant mass is at least partially mixed to obtain a further mixture;

    c. reacting the further mixture in the presence of the particulated porous catalyst to obtain a product stream.

11. A use of a reactor at least partially filled with a reactant mass, obtainable according to claims 1 to 8, claim 9, or both, for producing an unsaturated alkyl ester.

12. An unsaturated alkyl ester obtainable by the method according claim 10.

13. A use of an unsaturated alkyl ester obtainable by the method according to claim 10 for producing a polymer.

14. A method for producing a polymer comprising the steps

    a. providing a monomer obtainable by the method according to claim 10,
    b. polymerising the monomer to obtain the polymer.

15. A polymer obtainable by the method according to claim 14.

**Fig. 1**

EP 4 371 660 A1

**Fig. 2A**

<span style="float:right">200</span>

**Fig. 2B**

23

**Fig. 3**

300

```
┌─────────────┐
│     301     │
└─────────────┘
       ⇓
┌─────────────┐
│     302     │
└─────────────┘
       ⇓
┌─────────────┐
│     303     │
└─────────────┘
       ⇓
┌─────────────┐
│     304     │
└─────────────┘
```

**Fig. 4**

400

```
┌─────────────┐
│     401     │
└─────────────┘
       ⇓
┌─────────────┐
│     402     │
└─────────────┘
       ⇓
┌─────────────┐
│     403     │
└─────────────┘
```

## Fig. 5

**Fig. 6**

**Fig. 7**

Europäisches
Patentamt

European
Patent Office

Office européen
des brevets

# EUROPEAN SEARCH REPORT

Application Number

EP 22 20 8496

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/099731 A1 (LYGIN ALEXANDER [DE] ET AL) 4 April 2019 (2019-04-04) * paragraphs [0050] – [0052], [0055], [0062]; claims; figures; example 1 * | 1-15 | INV. B01J8/00 B01J8/08 B01J8/10 C08F220/18 C09J133/06 |
| A | US 2010/160460 A1 (SOTO JORGE L [US] ET AL) 24 June 2010 (2010-06-24) * claims; figure 1 * | 1-15 | |
| A | WO 2019/022892 A1 (ROHM & HAAS [US]; DOW GLOBAL TECHNOLOGIES LLC [US]) 31 January 2019 (2019-01-31) * page 4, line 29 – page 5, line 17; claims * | 1-15 | |
| A | FR 1 476 215 A (ROEHM & HAAS GMBH) 7 April 1967 (1967-04-07) * claims * | 13-15 | |

TECHNICAL FIELDS
SEARCHED      (IPC)

B01J
C08F
C09J

The present search report has been drawn up for all claims

| Place of search | Date of completion of the search | Examiner |
|---|---|---|
| Munich | 20 April 2023 | Serra, Renato |

EPO FORM 1503 03.82 (P04C01)

## ANNEX TO THE EUROPEAN SEARCH REPORT
## ON EUROPEAN PATENT APPLICATION NO.

EP 22 20 8496

This annex lists the patent family members relating to the patent documents cited in the above-mentioned European search report.
The members are as contained in the European Patent Office EDP file on
The European Patent Office is in no way liable for these particulars which are merely given for the purpose of information.

20-04-2023

| Patent document cited in search report | | Publication date | Patent family member(s) | | Publication date |
|---|---|---|---|---|---|
| US 2019099731 | A1 | 04-04-2019 | BR | 112018071599 A2 | 12-02-2019 |
| | | | CN | 109070036 A | 21-12-2018 |
| | | | EP | 3235560 A1 | 25-10-2017 |
| | | | EP | 3445483 A1 | 27-02-2019 |
| | | | JP | 6873155 B2 | 19-05-2021 |
| | | | JP | 2019514880 A | 06-06-2019 |
| | | | KR | 20180136958 A | 26-12-2018 |
| | | | RU | 2018140981 A | 22-05-2020 |
| | | | SA | 518400269 B1 | 06-12-2021 |
| | | | SG | 11201809192Y A | 29-11-2018 |
| | | | TW | 201806666 A | 01-03-2018 |
| | | | US | 2019099731 A1 | 04-04-2019 |
| | | | WO | 2017182381 A1 | 26-10-2017 |
| US 2010160460 | A1 | 24-06-2010 | AU | 2009330672 A1 | 21-07-2011 |
| | | | CN | 102292416 A | 21-12-2011 |
| | | | EP | 2379675 A1 | 26-10-2011 |
| | | | US | 2010160460 A1 | 24-06-2010 |
| | | | WO | 2010074762 A1 | 01-07-2010 |
| WO 2019022892 | A1 | 31-01-2019 | BR | 112020001603 A2 | 21-07-2020 |
| | | | CA | 3071244 A1 | 31-01-2019 |
| | | | CN | 110997615 A | 10-04-2020 |
| | | | EP | 3658531 A1 | 03-06-2020 |
| | | | JP | 7171697 B2 | 15-11-2022 |
| | | | JP | 2020528429 A | 24-09-2020 |
| | | | KR | 20200032713 A | 26-03-2020 |
| | | | SG | 11202000694W A | 27-02-2020 |
| | | | US | 2020157036 A1 | 21-05-2020 |
| | | | WO | 2019022892 A1 | 31-01-2019 |
| FR 1476215 | A | 07-04-1967 | NONE | | |

EPO FORM P0459

For more details about this annex : see Official Journal of the European Patent Office, No. 12/82